(19) Europäisches Patentamt
European Patent Office
Office européen des brevets

(11) Veröffentlichungsnummer: **0 128 381**
**A1**

# (12) EUROPÄISCHE PATENTANMELDUNG

(21) Anmeldenummer: **84105433.1**

(22) Anmeldetag: **14.05.84**

(51) Int. Cl.³: **C 07 C 79/355**, C 07 F 7/08, A 01 N 39/02, A 01 N 55/00

(30) Priorität: **27.05.83 DE 3319289**

(43) Veröffentlichungstag der Anmeldung: **19.12.84 Patentblatt 84/51**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Förster, Heinz, Dr., Am Eckbusch 47, D-5600 Wuppertal 1 (DE)**
Erfinder: **Gallenkamp, Bernd, Dr., Claudiusweg 5, D-5600 Wuppertal 1 (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert, R., Dr., Im Waldwinkel 110, D-5060 Bergisch Gladbach 2 (DE)**

## (54) Optisch aktive Phenoxypropionsäure-ester.

(57) Neue R-Enantiomere von Phenoxypropionsäureestern der Formel

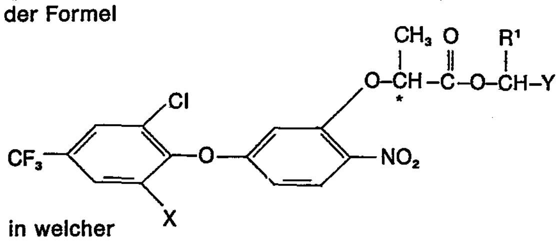

(I)

in welcher
X für Wasserstoff oder Halogen steht,
$R^1$ für Wasserstoff oder Alkyl steht und
Y für Trialkylsilyl, Alkoxyalkyl oder den Rest der Formel $-COOR^2$ steht, worin $R^2$ für Wasserstoff, Alkyl oder Alkoxyalkal steht,
mehrere Verfahren zur Herstellung der neuen Stoffe und deren Verwendung als Herbizide.

EP 0 128 381 A1

0128381

BAYER AKTIENGESELLSCHAFT 5090 Leverkusen, Bayerwerk

Konzernverwaltung RP
Patentabteilung Dü/ABc

Ib

# Optisch aktive Phenoxypropionsäure-ester

Die Erfindung betrifft neue R-Enantiomere*) von Phenoxypropionsäure-estern, mehrere Verfahren zu deren Herstellung und deren Verwendung als Herbizide.

Es ist bereits bekannt, daß zahlreiche Phenoxypropionsäure-ester herbizide Eigenschaften besitzen (vgl. DE-OS 29 06 087). So kann zum Beispiel das Racemat des 2-[3-(2,6-Dichlor-4-trifluor-methyl-phenoxy)-6-nitro-phenoxy]-propionsäure-(ethoxycarbonyl)-methylesters zur Bekämpfung von Unkraut eingesetzt werden. Die Wirkung dieses Produktes ist gut, jedoch werden bei sehr niedrigen Aufwandmengen einige Unkräuter nicht immer voll erfaßt.

---

*) Unter R-Enantiomeren sind im vorliegenden Fall jeweils diejenigen optisch aktiven Verbindungen zu verstehen, welche am asymmetrischen Kohlenstoffatom der Propionsäure-Einheit die R-Konfiguration aufweisen.

Es wurden jetzt neue R-Enantiomere von Phenoxypropionsäure-estern der Formel

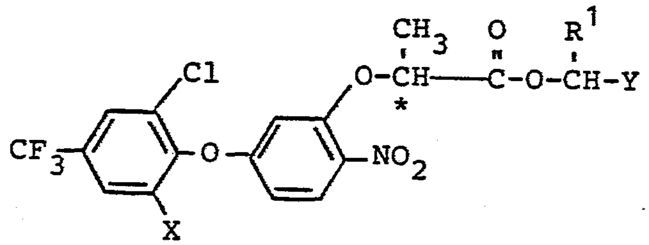

(I)

in welcher

X für Wasserstoff oder Halogen steht,

$R^1$ für Wasserstoff oder Alkyl steht und

Y für Trialkylsilyl, Alkoxyalkyl oder den Rest der Formel $-COOR^2$ steht, worin $R^2$ für Wasserstoff, Alkyl oder Alkoxyalkyl steht,

gefunden.

Weiterhin wurde gefunden, daß man die neuen R-Enantiomeren von Phenoxypropionsäureestern der Formel (I) erhält, wenn man

a) R-Enantiomere von Phenoxypropionsäure-Chloriden der Formel

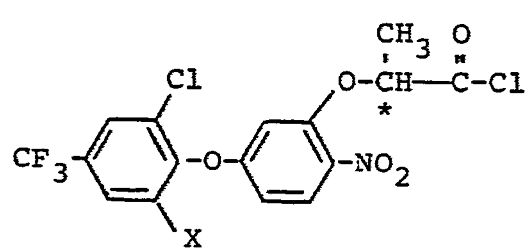

(II)

in welcher

X die oben angegebene Bedeutung hat,

mit Hydroxy-Verbindungen der Formel

$$HO-\underset{}{\overset{\displaystyle R^1}{\overset{|}{C}}}H-Y^1 \qquad (III)$$

in welcher

$R^1$ die oben angegebene Bedeutung hat und

$Y^1$ für Trialkylsilyl, Alkoxyalkyl oder den Rest der Formel $-COOR^3$ steht, worin
$R^3$ für Alkyl oder Alkoxyalkyl steht,

gegebenenfalls in Gegenwart eines Säureakzeptors sowie gegebenenfalls in Gegenwart eines Verdünnungsmittels umsetzt, und gegebenenfalls dabei entstehende Ester in Gegenwart eines Verdünnungsmittels verseift,

oder

b) Diphenylether-Derivate der Formel

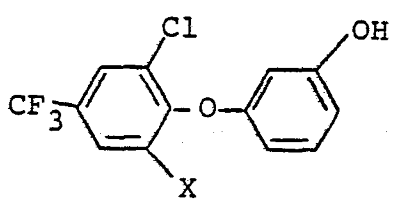

(IV)

in welcher

X die oben angegebene Bedeutung hat,

mit S-Enantiomeren der Propionsäure-Derivate der Formel

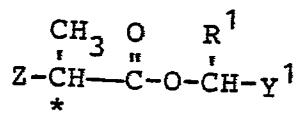

(V)

in welcher

$R^1$ und $Y^1$ die oben angegebene Bedeutung haben und

Z für Tosylat oder Mesylat steht,

gegebenenfalls in Gegenwart eines Säureakzeptors sowie gegebenenfalls in Gegenwart eines Verdünnungsmittels umsetzt und anschließend die dabei entstehenden R-Enantiomeren der Diphenylether-Derivate der Formel

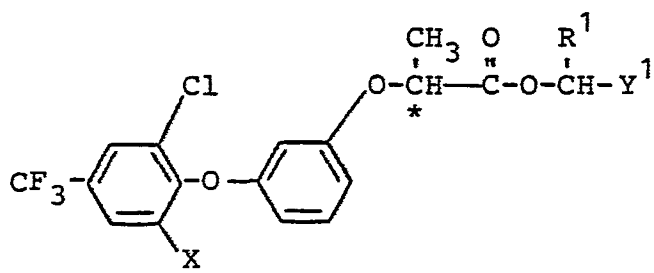

(VI)

in welcher

$R^1$, X und $Y^1$ die oben angegebene Bedeutung haben,

mit einem Nitrierungsmittel, gegebenenfalls in Gegenwart eines Katalysators sowie gegebenenfalls in Gegenwart eines Verdünnungsmittels umsetzt und gegebenenfalls dabei entstehende Ester in Gegenwart eines Verdünnungsmittels verseift,

oder

c) R-Enantiomere von Phenoxypropionsäure-Derivaten der Formel

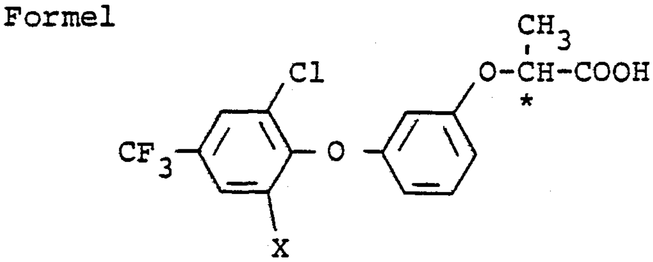

(VII)

in welcher

X die oben angegebene Bedeutung hat,

mit Halogen-Verbindungen der Formel

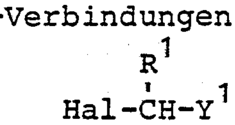

(VIII)

in welcher

$R^1$ und $Y^1$ die oben angegebene Bedeutung haben und

Hal für Chlor oder Brom steht,

in Gegenwart eines Säureakzeptors sowie gegebenenfalls in Gegenwart eines Verdünnungsmittels umsetzt und anschließend die dabei entstehenden R-Enantiomeren der Diphenylether-Derivate der Formel

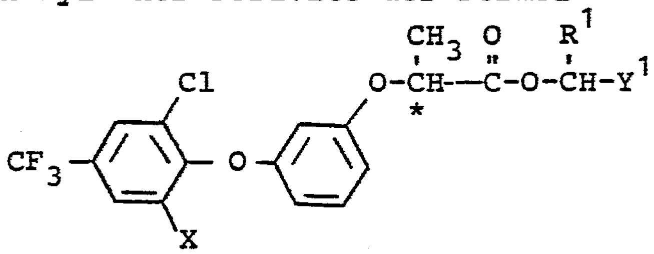

(VI)

in welcher

$R^1$, X und $Y^1$ die oben angegebene Bedeutung haben,

mit einem Nitrierungsmittel, gegebenenfalls in Gegenwart eines Katalysators sowie gegebenenfalls in Gegenwart eines Verdünnungsmittels umsetzt und gegebenenfalls dabei entstehende Ester in Gegenwart eines Verdünnungsmittels verseift.

Schließlich wurde gefunden, daß sich die neuen R-Enantiomeren von Phenoxypropionsäure-estern der Formel (I) durch eine hervorragende herbizide Wirksamkeit auszeichnen.

Überraschenderweise besitzen die erfindungsgemäßen R-Enantiomeren der Phenoxypropionsäure-ester der Formel (I) wesentlich bessere herbizide Eigenschaften als die Racemate dieser Stoffe. So übertrifft zum Beispiel das R-Enantiomere des 2-/3-(2,6-Dichlor-4-trifluormethyl-phenoxy)-6-nitro-phenoxy7-propionsäure-(ethoxycarbonyl)-ethylesters in seiner herbiziden Wirksamkeit das entsprechende Racemat, das aus dem Stand der Technik als gut wirksames Herbizid bekannt ist.

Die erfindungsgemäßen optisch aktiven Verbindungen sind durch die Formel (I) allgemein definiert. In dieser Formel in der das asymmetrische Kohlenstoffatom durch ein (*) gekennzeichnet ist, steht X vorzugsweise für Wasserstoff oder Chlor. $R^1$ steht für Wasserstoff oder für geradkettiges oder verzweigtes Alkyl mit 1 bis 4 Kohlenstoffatomen, insbesondere für Wasserstoff, Methyl oder Ethyl. Der Rest Y steht vorzugsweise für Trialkylsilyl mit 1 bis 4 Kohlenstoffatomen in jeder Alkylgruppe, insbesondere mit 1 oder 2 Kohlenstoffatomen in jeder Alkylgruppe. Weiterhin steht Y vorzugsweise für Alkoxyalkyl mit 1 bis 4 Kohlenstoffatomen in der Alkoxygruppe und 1 bis 4 Kohlenstoffatomen in der Alkylgruppe, wobei Methoxymethyl, Methoxyethyl, Ethoxymethyl und Ethoxyethyl speziell genannt seien. Ferner steht Y für den Rest der Formel $-COOR^2$, worin $R^2$ vorzugsweise für Wasserstoff, Alkyl mit 1 bis 4 Kohlenstoffatomen oder für Alkoxyalkyl mit 1 bis 4 Kohlenstoffatomen in der Alkoxygruppe und 1 bis 4 Kohlenstoffatomen in der Alkylgruppe steht. Insbesondere steht $R^2$ für Wasserstoff, Methyl, Ethyl, n-Propyl, iso-Propyl, n-Butyl, iso-Butyl, sek.-Butyl, tert.-Butyl, Methoxymethyl, Methoxyethyl, Ethoxymethyl oder Ethoxyethyl.

Diejenigen R-Enantiomeren der Formel (I), in denen $R^1$ für Alkyl steht, enthalten ein zweites Asymmetriezentrum in der Ester-Einheit. Die betreffenden Stoffe können daher in den folgenden Formen vorliegen:

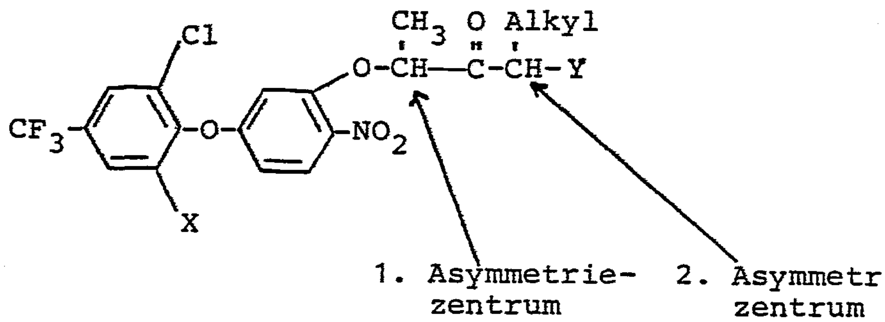

(Ia)

1. Asymmetriezentrum 2. Asymmetriezentrum

| Konfiguration am 1. Asymmetriezentrum | Konfiguration am 2. Asymmetriezentrum |
|---|---|
| R | R |
| R | S |
| R | R/S (Racemat) |

Die vorliegende Erfindung umfaßt sowohl diejenigen Verbindungen, die am zweiten Asymmetriezentrum die R- oder die S-Konfiguration aufweisen als auch die entsprechenden Racemate.

Verwendet man das R-Enantiomere des 2-/3-(2-Chlor-4-trifluormethyl-phenoxy)-6-nitrophenoxy7-propionylchlorids und Hydroxyessigsäure-ethylester als Ausgangsstoffe, so kann der Verlauf des erfindungsgemäßen Verfahrens (a) durch das folgende Formelschema wiedergegeben werden:

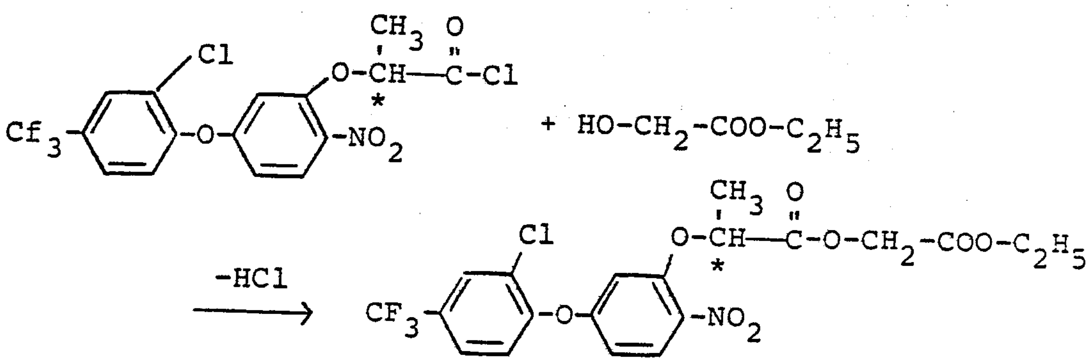


Verwendet man 2,6-Dichlor-4-trifluormethyl-3'-hydroxy-diphenylether und das S-Enantiomere des 2-Tosyloxy-propionsäure-(ethoxycarbonyl)-methylesters als Ausgangsstoffe und konzentrierte Salpetersäure als Nitrierungsmittel, so kann der Verlauf des erfindungsgemäßen Verfahrens (b) durch das folgende Formelschema wiedergegeben werden:

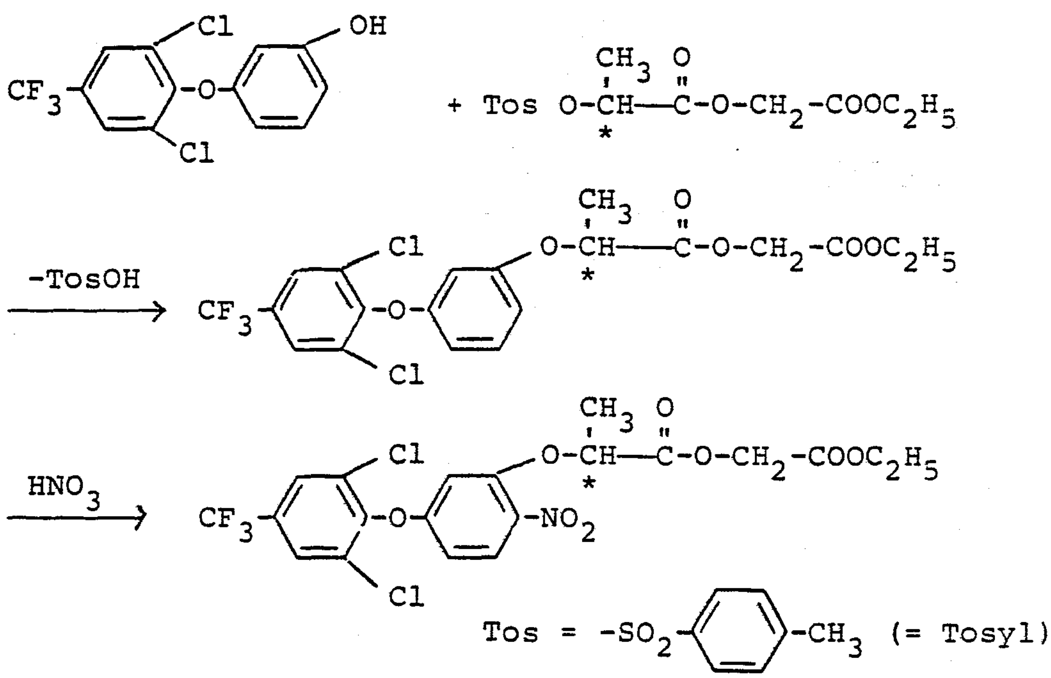

Verwendet man das R-Enantiomere der 2-[3-(2,6-Dichlor-4-trifluormethyl-phenoxy)-phenoxy]-propionsäure und Bromessigsäure-ethylester als Ausgangsstoffe und Kupfernitrat als Nitrierungsmittel, so kann der Verlauf des erfindungsgemäßen Verfahrens (c) durch das folgende Formelschema wiedergegeben werden:

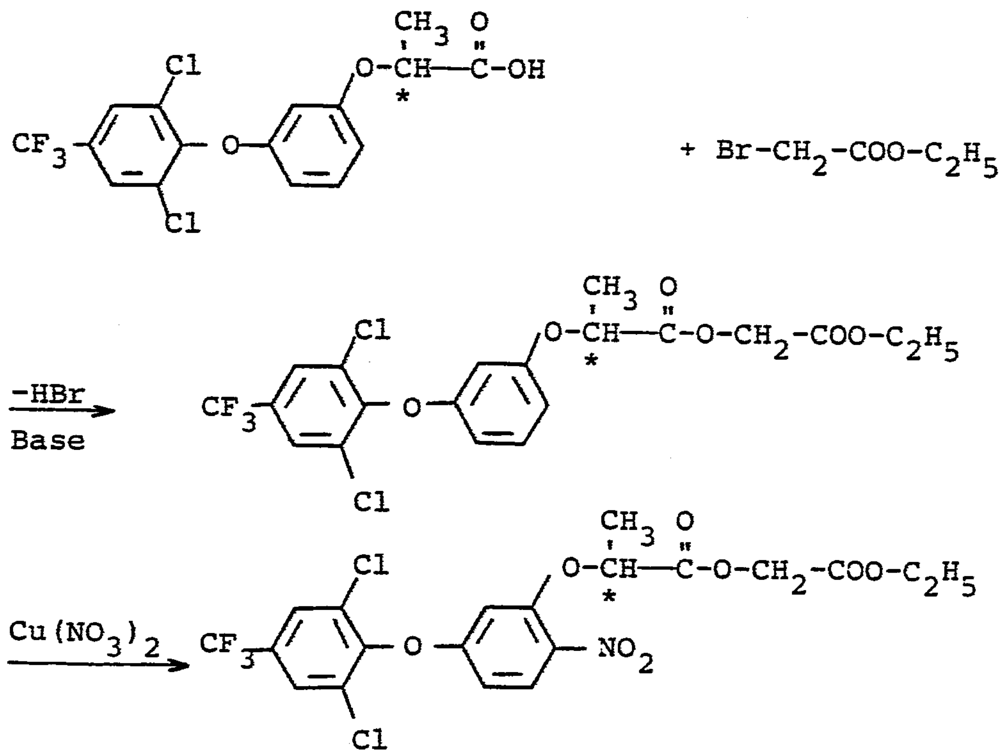

Die bei dem erfindungsgemäßen Verfahren (a) als Ausgangsstoffe benötigten R-Enantiomeren von Phenoxypropionsäurechloriden sind durch die Formel (II) definiert. In dieser Formel steht X vorzugsweise für Wasserstoff oder Chlor.

Die R-Enantiomeren von Phenoxypropionsäurechloriden der Formel (II) sind bisher noch nicht bekannt. Sie lassen sich jedoch in einfacher Weise herstellen, indem man R-Enantiomere von Phenoxypropionsäuren der Formel

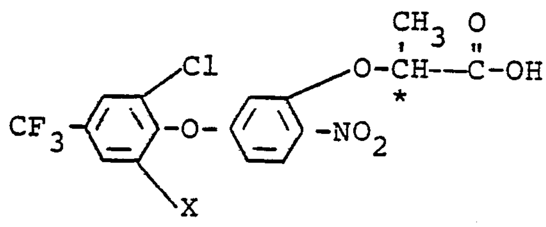

(IX)

in welcher

X die oben angegebene Bedeutung hat,

mit Chlorierungsmitteln, wie zum Beispiel Thionylchlorid, gegebenenfalls in Gegenwart eines Verdünnungsmittels, wie zum Beispiel Methylenchlorid oder 1,2-Dichlorethan, bei Temperaturen zwischen 10 und 100°C umsetzt und destillativ leicht flüchtige Komponenten nach dem Ende der Reaktion entfernt.

Die R-Enantiomeren der Phenoxypropionsäuren der Formel (IX) sind ebenfalls noch nicht bekannt. Sie lassen sich herstellen, indem man

in einer <u>ersten Stufe</u> Diphenylether der Formel

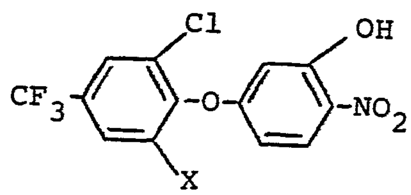

(X)

in welcher

X die oben angegebene Bedeutung hat,

mit S-Enantiomeren der Propionsäure-Derivate der Formel

$$Z-\underset{*}{\overset{CH_3}{CH}}-COO-R \qquad (XI)$$

in welcher

Z die oben angegebene Bedeutung hat und

R für Methyl, Ethyl oder Propyl steht,

gegebenenfalls in Gegenwart eines Säureakzeptors, wie zum Beispiel Kaliumcarbonat oder Triethylamin, sowie gegebenenfalls in Gegenwart eines Verdünnungsmittels, wie zum Beispiel Acetonitril, bei Temperaturen zwischen 0°C und 140°C umsetzt und

in einer <u>zweiten Stufe</u> die entstandenen R-Enantiomeren der Phenoxypropionsäure-ester der Formel

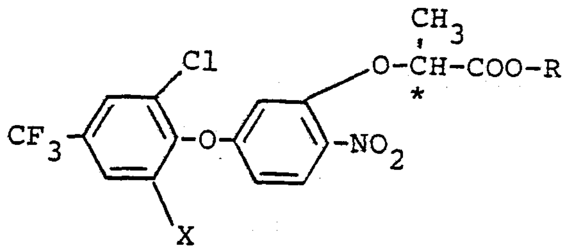

(XII)

in welcher
R und X die oben angegebene Bedeutung haben,

mit wäßrigen Alkalilaugen, wie zum Beispiel Natronlauge oder Kalilauge, gegebenenfalls in Gegenwart eines organischen Verdünnungsmittels, wie zum Beispiel Methanol, Ethanol, Dioxan oder Xylol, bei Temperaturen zwischen 20 und 120°C umsetzt und aus den anfallenden Salzen durch Ansäuern, zum Beispiel mit Salzsäure die gewünschten Säuren freisetzt.

Die bei der Durchführung des obigen Verfahrens als Ausgangsstoffe benötigten Diphenylether der Formel (X) sind bekannt (vgl. DE-OS 29 06 087).

Die als Reaktionskomponenten benötigten S-Enantiomeren der Propionsäure-Derivate der Formel (XI) sind ebenfalls bekannt oder lassen sich in einfacher Weise nach bekannten Methoden herstellen.

Bei der oben beschriebenen Herstellung der R-Enantiomeren der Phenoxypropionsäuren der Formel (IX) ist der Einsatz von S-Enantiomeren der Propionsäure-Derivate der Formel (XI) erforderlich, da im Verlauf der Umsetzung am asymmetrischen Kohlenstoffatom eine Walden'sche Umkehr erfolgt. Die S-Enantiomeren der Propionsäure-Derivate der Formel (XI)

drehen die Ebene des linear polarisierten Lichtes nach links. Den gleichen Drehsinn besitzen auch die R-Enantiomeren von Phenoxypropionsäuren der Formel (IX), obwohl sich die Konfiguration am asymmetrischen Kohlenstoffatom im Zuge der Umsetzung umgekehrt hat.

Die bei dem erfindungsgemäßen Verfahren (a) als Reaktionskomponenten benötigten Hydroxy-Verbindungen sind durch die Formel (III) definiert. In dieser Formel steht $R^1$ vorzugsweise für Wasserstoff oder für geradkettiges oder verzweigtes Alkyl mit 1 bis 4 Kohlenstoffatomen, insbesondere für Wasserstoff, Methyl oder Ethyl. Der Rest $Y^1$ steht vorzugsweise für Trialkylsilyl mit 1 bis 4 Kohlenstoffatomen in jeder Alkylgruppe, insbesondere mit 1 oder 2 Kohlenstoffatomen in jeder Alkylgruppe. Weiterhin steht $Y^1$ vorzugsweise für Alkoxyalkyl mit 1 bis 4 Kohlenstoffatomen in der Alkoxygruppe und 1 bis 4 Kohlenstoffatomen in der Alkylgruppe, wobei Methoxymethyl, Methoxyethyl, Ethoxymethyl und Ethoxyethyl speziell genannt seien. Ferner steht $Y^1$ für den Rest der Formel $-COOR^3$, worin $R^3$ vorzugsweise für Alkyl mit 1 bis 4 Kohlenstoffatomen oder für Alkoxyalkyl mit 1 bis 4 Kohlenstoffatomen in der Alkoxygruppe und 1 bis 4 Kohlenstoffatomen in der Alkylgruppe steht. Insbesondere steht $R^3$ für Methyl, Ethyl, n-Propyl, iso-Propyl, n-Butyl, iso-Butyl, sek.-Butyl, tert.-Butyl, Methoxymethyl, Methoxyethyl, Ethoxymethyl oder Ethoxyethyl.

Diejenigen Stoffe der Formel (III), in denen $R^1$ nicht für Wasserstoff steht, können entweder als Racemate oder als

R- bzw. S-Enantiomere vorliegen. Verwendet man bei dem erfindungsgemäßen Verfahren (a) die R-Enantiomeren der Hydroxy-Verbindungen der Formel (III), so entstehen diejenigen erfindungsgemäßen Stoffe, die am zweiten Asymmetriezentrum (vgl. oben) die R-Konfiguration aufweisen. Entsprechend entstehen aus den S-Enantiomeren diejenigen erfindungsgemäßen Stoffe, die am zweiten Asymmetriezentrum die S-Konfiguration besitzen. Bei Verwendung von Racematen der Hydroxy-Verbindungen der Formel (III) fallen erfindungsgemäße Stoffe an, in denen das asymmetrisch substituierte Kohlenstoffatom im Esterteil keinen Beitrag zur optischen Aktivität der Endprodukte leistet.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalihydroxide, Erdalkalihydroxide und -oxide, wie z.B. Natrium- und Kaliumhydroxid, Calciumhydroxid und Calciumoxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzyl-amin, 1,5-Diazabicyclo/4,3,0/non-5-en (DBN), 1,8-Diazabicyclo/5,4,0/-undec-7-en (DBU) und Pyridin.

Als Verdünnungsmittel kommen bei der Durchführung des erfindungsgemäßen Verfahrens (a) alle inerten organischen Solventien in Betracht. Vorzugsweise verwendbar sind aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methylisopropyl-, und Methyl-isobutyl-keton, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperaturen können bei der Umsetzung nach dem erfindungsgemäßen Verfahren (a) innerhalb eines größeren Bereiches variiert werden. Im allgemeinen arbeitet man bei Temperaturen zwischen -20°C und +160°C, vorzugsweise zwischen 0°C und +140°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die Ausgangsstoffe der Formeln (II) und (III) im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere

Stunden bei der jeweils erforderlichen Temperatur gerührt. Die Aufarbeitung erfolgt jeweils nach üblichen Verfahren. Im allgemeinen geht man so vor, daß man das Reaktionsgemisch mit Wasser versetzt, mehrfach mit einem mit Wasser wenig mischbaren organischen Lösungsmittel extrahiert, die vereinigten organischen Phasen nacheinander mit verdünnter wäßriger Alkalilauge, verdünnter wäßriger Salzsäure und Wasser wäscht, dann trocknet und durch Abziehen des Lösungsmittels unter vermindertem Druck einengt.

Ist die Herstellung einer Verbindung der Formel (I) beabsichtigt, in der Y für den Carboxylrest der Formel COOH steht, so ist eine Verseifung eines entsprechenden Esters erforderlich. Diese Verseifung erfolgt nach üblichen Methoden. Vorzugsweise verwendet man wäßrige Alkalihydroxidlaugen, wie zum Beispiel Natronlauge oder Kalilauge, als Verseifungsreagenzien.

Die Esterverseifung wird im allgemeinen in Gegenwart eines Verdünnungsmittels vorgenommen. Vorzugsweise in Betracht kommen aromatische Kohlenwasserstoffe, wie Toluol oder Xylol, ferner Alkohole, wie Methanol oder Ethanol, außerdem Ether, wie Dioxan, weiterhin Nitrile, wie Acetonitril, und auch Gemische aus organischen Solventien und Wasser.

Die Temperaturen können bei der Durchführung der Esterverseifung innerhalb eines größeren Bereiches variiert werden. Im allgemeinen arbeitet man bei Temperaturen zwischen 0°C und 160°C, vorzugsweise zwischen 20°C und 140°C.

Bei der Durchführung der Esterverseifung geht man im allgemeinen so vor, daß man den Ester der Formel (I) in Gegenwart eines Verdünnungsmittels mit einer äquivalenten Menge oder mit einem Überschuß an Base bei der jeweils gewünschten Temperatur behandelt. Die Aufarbeitung erfolgt nach üblichen Methoden. Im allgemeinen geht man so vor, daß das Reaktionsgemisch durch Abziehen des Lösungsmittels unter vermindertem Druck einengt, den verbleibenden Rückstand in Wasser aufnimmt, mit Mineralsäure, wie zum Beispiel Salzsäure, ansäuert und die sich dabei abscheidende Säure der Formel (I) abtrennt.

Die bei der Durchführung des erfindungsgemäßen Verfahrens (b) als Ausgangsstoffe benötigten Diphenylether-Derivate sind durch die Formel (IV) definiert. In dieser Formel steht X vorzugsweise für Wasserstoff oder Chlor.

Die Diphenylether-Derivate der Formel (IV) sind bekannt (vgl. DE-OS 28 05 981).

Die bei dem erfindungsgemäßen Verfahren (b) weiterhin als Ausgangsstoffe benötigten S-Enantiomeren der Propionsäure-Derivate sind durch die Formel (V) definiert. In dieser Formel steht $R^1$ vorzugsweise für Wasserstoff oder für geradkettiges oder verzweigtes Alkyl mit 1 bis 4 Kohlenstoffatomen, insbesondere für Wasserstoff, Methyl oder Ethyl. Der Rest $Y^1$ steht vorzugsweise für Trialkylsilyl mit 1 bis 4 Kohlenstoffatomen in jeder Alkylgruppe, insbesondere mit 1 oder 2 Kohlenstoffatomen in jeder

Alkylgruppe. Weiterhin steht $Y^1$ vorzugsweise für Alkoxyalkyl mit 1 bis 4 Kohlenstoffatomen in der Alkoxygruppe und 1 bis 4 Kohlenstoffatomen in der Alkylgruppe, wobei Methoxymethyl, Methoxyethyl, Ethoxymethyl und Ethoxyethyl speziell genannt seien. Ferner steht $Y^1$ für den Rest der Formel $-COOR^3$, worin $R^3$ vorzugsweise für Alkyl mit 1 bis 4 Kohlenstoffatomen oder für Alkoxyalkyl mit 1 bis 4 Kohlenstoffatomen in der Alkoxygruppe und 1 bis 4 Kohlenstoffatomen in der Alkylgruppe steht. Insbesondere steht $R^3$ für Methyl, Ethyl, n-Propyl, iso-Propyl, n-Butyl, iso-Butyl, sek.-Butyl, tert.-Butyl, Methoxymethyl, Methoxyethyl, Ethoxymethyl oder Ethoxyethyl. Z steht in der Formel (V) für Tosylat

($O-SO_2$- 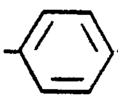 -$CH_3$) oder Mesylat ($-O-SO_2-CH_3$).

Diejenigen Stoffe der Formel (V), in denen $R^1$ nicht für Wasserstoff steht, können wiederum im Ester-Teil ein zweites Asymmetriezentrum enthalten, das in der R- oder in der S-Konfiguration vorliegen kann. In den entsprechenden Racematen leistet das asymmetrisch substituierte Kohlenstoffatom im Ester-Teil keinen Beitrag zur optischen Aktivität der S-Enantiomeren der Propionsäure-Derivate der Formel (V).

Die S-Enantiomeren der Propionsäure-Derivate der Formel (V) sind bekannt oder lassen sich nach üblichen Methoden in einfacher Weise herstellen. So erhält man die S-Enantiomeren der Propionsäure-Derivate der Formel (V), indem man S-Enantiomere von Propionylchloriden der Formel

$$\begin{array}{c} CH_3 \\ | \\ Z-\underset{*}{C}-CO-Cl \end{array} \qquad (XIII)$$

in welcher

Z die oben angegebene Bedeutung hat,

mit Hydroxy-Verbindungen der Formel

$$\begin{array}{c} R^1 \\ | \\ HO-CH-Y^1 \end{array} \qquad (III)$$

in welcher

$R^1$ und $Y^1$ die oben angegebene Bedeutung haben,

gegebenenfalls in Gegenwart eines Säureakzeptors sowie gegebenenfalls in Gegenwart eines Verdünnungsmittels umsetzt.

Bei diesem Verfahren zur Herstellung der S-Enantiomeren der Propionsäure-Derivate der Formel (V) entsprechen die Umsetzungsbedingungen denen des erfindungsgemäßen Verfahrens (a).

Der Einsatz von S-Enantiomeren der Phenoxypropionsäure-Derivate der Formel (V) ist bei dem erfindungsgemäßen Verfahren (b) deshalb erforderlich, weil im Verlauf der Reaktion eine Walden'sche Umkehr am asymmetrisch substituierten Kohlenstoffatom der Propionsäure-Einheit erfolgt.

Zur Nitrierung der R-Enantiomeren der Diphenylether der Formel (VI) verwendet man im Falle des erfindungsgemäßen Verfahrens (b) vorzugsweise konzentrierte Salpetersäure oder Schwermetall-Nitrate, wie zum Beispiel Eisen(III)-nitrat oder Kupfernitrat.

Die erste Stufe des erfindungsgemäßen Verfahrens (b) wird vorzugsweise in Gegenwart eines Säureakzeptors und eines Verdünnungsmittels durchgeführt. Hierbei kommen als Säurebindemittel und Verdünnungsmittel alle diejenigen Stoffe in Betracht, die bereits im Zusammenhang mit der Beschreibung des erfindungsgemäßen Verfahrens (a) genannt wurden.

Als Katalysatoren für die in der zweiten Stufe des erfindungsgemäßen Verfahrens (b) durchzuführende Nitrierung kommen vorzugsweise Protonensäuren, wie zum Beispiel Schwefelsäure oder Essigsäure, in Betracht.

Als Verdünnungsmittel kommen bei der Durchführung der zweiten Stufe des erfindungsgemäßen Verfahrens (b) vorzugsweise halogenierte Kohlenwasserstoffe, wie zum Beispiel Methylenchlorid oder 1,2-Dichlorethan, und ferner Carbonsäureanhydride, wie zum Beispiel Essigsäureanhydrid, in Frage.

Die Reaktionstemperaturen können bei dem erfindungsgemäßen Verfahren (b) innerhalb eines größeren Bereiches variiert werden. Im allgemeinen arbeitet man in der ersten Stufe bei Temeraturen zwischen -20°C, und +160°C, vorzugsweise zwischen 0°C und +140°C. In der zweiten Stufe arbeitet

man im allgemeinen bei Temperaturen zwischen -20°C und +60°C, vorzugsweise zwischen 0°C und +40°C.

Das erfindungsgemäße Verfahren (b) wird im allgemeinen unter Normaldruck durchgeführt. Es ist jedoch auch möglich, unter erhöhtem oder vermindertem Druck zu arbeiten.

Die Durchführung des erfindungsgemäßen Verfahrens (b) erfolgt in der ersten Stufe so, wie es bereits für das erfindungsgemäße Verfahren (a) beschrieben wurde. Zur Durchführung der zweiten Stufe des erfindungsgemäßen Verfahrens (b) setzt man auf 1 Mol einer Verbindung der Formel (VI) im allgemeinen 0,9 bis 2 Mol, vorzugsweise 1,0 bis 1,3 Mol an Nitrierungsmittel und gegebenenfalls etwa die gleiche Menge eines Katalysators ein. Die Ausgangskomponenten werden vorzugsweise unter Eiskühlung zusammengegeben und dann gegebenenfalls bei leicht erhöhter Temperatur bis zum Ende der Umsetzung gerührt.

Die Aufarbeitung erfolgt nach üblichen Methoden. Im allgemeinen verfährt man in der Weise, daß man das Reaktionsgemisch nach beendeter Umsetzung in Eiswasser gießt, absaugt bzw. extrahiert und das anfallende Produkt gegebenenfalls durch Umkristallisieren reinigt.

Wenn nach dem erfindungsgemäßen Verfahren (b) diejenigen Verbindungen der Formel (I) hergestellt werden sollen, in denen Y für COOH steht, dann führt man die Verseifung der entsprechenden Ester so durch, wie es bereits im Falle des erfindungsgemäßen Verfahrens (a) beschrieben wurde.

Die bei dem erfindungsgemäßen Verfahren (c) als Ausgangsstoffe benötigten R-Enantiomeren von Phenoxypropionsäure-Derivaten sind durch die Formel (VII) definiert. In dieser Formel steht X vorzugsweise für Wasserstoff oder Chlor.

Die Verbindungen der Formel (VII) sind bisher noch nicht bekannt. Sie lassen sich herstellen, indem man Diphenylether-Derivate der Formel

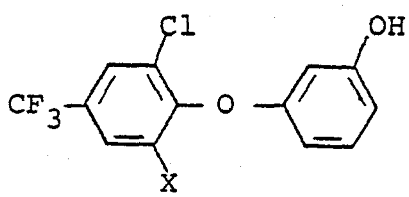

(IV)

in welcher
X die oben angegebene Bedeutung hat,

mit S-Enantiomeren der Propionsäure-Derivate der Formel

$$\underset{*}{\overset{CH_3}{\underset{|}{Z-CH-COO-R}}}$$

(XI)

in welcher
Z die oben angegebene Bedeutung hat und
R für Methyl, Ethyl oder Propyl steht,

gegebenenfalls in Gegenwart eines Säureakzeptors, wie zum Beispiel Kaliumcarbonat oder Triethylamin, sowie gegebenenfalls in Gegenwart eines Verdünnungsmittels, wie zum Beispiel Acetonitril, bei Temperaturen zwischen 0°C und 140°C umsetzt und anschließend verseift.

Die bei dem erfindungsgemäßen Verfahren (c) weiterhin als Ausgangsstoffe benötigten Halogen-Verbindungen sind durch die Formel (VIII) definiert. In dieser Formel haben $R^1$ und X vorzugsweise diejenigen Bedeutungen, die oben bereits als vorzugsweise in Frage kommende Bedeutungen für diese Reste genannt wurden. Hal steht vorzugsweise für Chlor oder Brom.

Die Halogen-Verbindungen der Formel (VIII) sind bekannt oder lassen sich nach bekannten Methoden in einfacher Weise herstellen.

Zur Nitrierung der R-Enantiomeren der Diphenylether-Derivate der Formel (VI) verwendet man im Falle des erfindungsgemäßen Verfahrens (c) ebenso wie bei dem erfindungsgemäßen Verfahren (b) vorzugsweise konzentrierte Salpetersäure oder Schwermetall-Nitrate, wie zum Beispiel Eisen(III)nitrat oder Kupfernitrat.

Die erste Stufe des erfindungsgemäßen Verfahrens (c) wird vorzugsweise in Gegenwart eines Säureakzeptors und eines Verdünnungsmittels durchgeführt. Hierbei kommen als Säurebindemittel und als Verdünnungsmittel alle diejenigen Stoffe in Betracht, die in diesem Zusammenhang bereits im Falle des erfindungsgemäßen Verfahrens (a) genannt wurden.

Die Nitrierung erfolgt im Falle des erfindungsgemäßen Verfahrens (c) in gleicher Weise wie im Falle des erfindungsgemäßen Verfahrens (b). Ebenso entsprechen die übrigen Reaktionsbedingungen, wie Temperaturen und Molverhältnisse der Reaktionskomponenten, denen des erfindungsgemäßen Verfahrens (b). Auch die Aufarbeitung und die gegebenenfalls durchzuführende abschließende Verseifung werden so vorgenommen wie es bereits im Falle des erfindungsgemäßen Verfahrens (b) erwähnt wurde.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

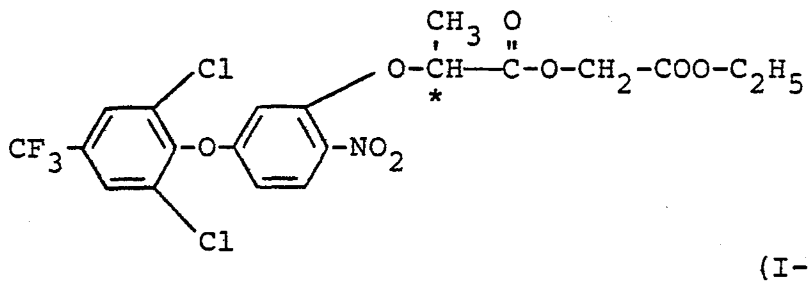

(I-1)

a) Verfahrensvariante (a):

Eine Lösung von 30 g (0,06 Mol) des R-Enantiomeren des 2-[3-(2,6-Dichlor-4-trifluormethyl-phenoxy)-6-nitro-phenoxy]-propionylchlorids in 100 ml Toluol wurde bei Temperaturen zwischen 0°C und +5°C in ein Gemisch aus 7,4 g (0,071 Mol) Hydroxy-essigsäure-ethylester und 4 g (0,08 Mol) Triethylamin in 160 ml Toluol gegeben. Man rührte weitere 14 Stunden bei Raumtemperatur und arbeitete dann auf, indem man das Reaktionsgemisch zunächst mit Wasser versetzte, mehrfach mit Toluol extrahierte, die vereinigten organischen Phasen nacheinander mit verdünnter wäßriger Natronlauge, verdünnter wäßriger Salzsäure und Wasser wusch, dann trocknete und durch Abziehen des Lösungsmittels unter vermindertem Druck einengte. Man erhielt auf diese Weise 25 g (81 % der Theorie) an dem R-Enantiomeren des 2-[3-(2,6-Dichlor-4-trifluormethyl-phenoxy)-6-nitrophenoxy]-propionsäure-(ethoxycarbonyl)-methylesters.

Drehwert: $[\alpha]_D^{24} = -10,6°$

(1-molare Lösung in Chloroform; Küvettenlänge = 10 cm).

<u>Herstellung von Ausgangsprodukten</u>

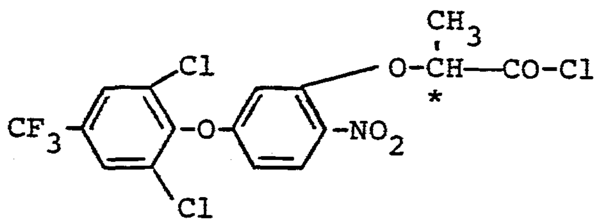

(II-1)

Eine Lösung von 35 g (0,079 Mol) des R-Enantiomeren der 2-[3-(2,6-Dichlor-4-trifluormethyl-phenoxy)-6-nitro-phenoxy]- Propionsäure und 0,58 ml Dimethylformamid in 150 ml Methylenchlorid wurde bei Raumtemperatur unter Rühren tropfenweise mit 11,3 g (0,095 Mol) Thionylchlorid versetzt und danach 4 Stunden unter Rückfluß erhitzt. Anschließend wurde das Reaktionsgemisch durch Abziehen des Lösungsmittels unter vermindertem Druck eingeengt. Man erhielt auf diese Weise 32 g (88,4 % der Theorie) an dem R-Enantiomeren des 2-[3-(2,6-Dichlor-4-trifluormethyl-phenoxy)-6-nitrophenoxy]-propionylchlorids.

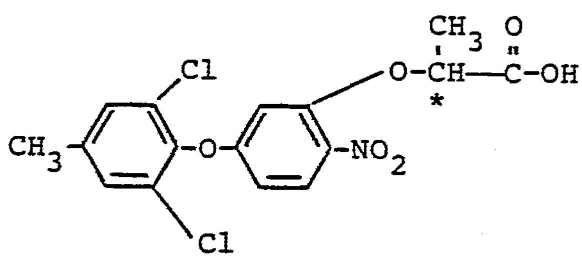

(IX-1)

Eine Lösung von 17,6 g (0,44 Mol) Natriumhydroxid in 25 ml Wasser wurde unter schnellem Rühren in eine auf 120°C erhitzte Lösung von 206 g (0,44 Mol) des R-Enantiomeren des 2-[3-(2,6-Dichlor-4-trifluormethyl-phenoxy)-6-nitro-phenoxy]-propionsäure-ethylesters in 500 ml Xylol langsam eingetropft. Man rührte noch weitere 1,5 Stunden bei

120°C und arbeitete dann auf, indem man das Lösungsmittel abdestillierte, den verbleibenden Rückstand mit Wasser aufnahm, dann mit 50 ml konzentrierter Salzsäure ansäuerte und das sich abscheidende Produkt abtrennte. Man erhielt auf diese Weise 70 g (36,2 % der Theorie) an R-Enantiomerem der 2-/3-(2,6-Dichlor-4-trifluormethyl-phenoxy)-6-nitro-phenoxy/-propionsäure.

Drehwert: $[\alpha]_D^{24}$ = -20,4°
(1-molare Lösung in Chloroform; Küvettenlänge 10 cm)

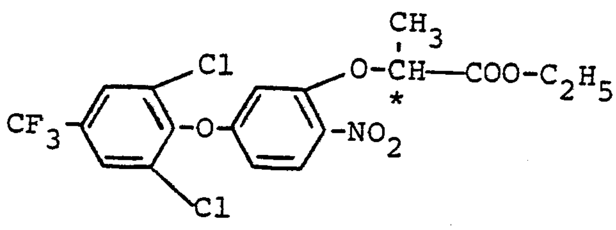

(XII-1)

Ein Gemisch aus 36,8 g (0,1 Mol) 2,6-Dichlor-4-trifluormethyl-3'-hydroxy-4'-nitro-diphenylether, 28,5 g (0,1 Mol) an S-Enantiomerem des 2-Tosyloxy-propionsäureethylesters und 28 g (0,2 Mol) Kaliumcarbonat in 200 ml Acetonitril wurde 15 Stunden unter Rückfluß erhitzt. Danach wurde aufgearbeitet, indem man das Reaktionsgemisch in Wasser gab, mehrfach mit Toluol extrahierte, die vereinigten organischen Phasen nacheinander mit Wasser, verdünnter wäßriger Natronlauge und wieder mit Wasser wusch, dann trocknete und durch Abziehen des Lösungsmittels unter vermindertem Druck einengte. Der verbleibende Rückstand wurde durch leichtes Erwärmen im Hochvakuum von Resten an flüchtigen Anteilen befreit. Man erhielt auf diese Weise 22 g (47 % der Theorie) an R-Enantiomerem

des 2-[3-(2,6-Dichlor-4-trifluormethyl-phenoxy)-6-nitro-phenoxy]-propionsäure-ethylesters.

Drehwert: $[\alpha]_D^{24}$ = - 14,4°
(1-molare Lösung in Chloroform; Küvettenlänge 10 cm)

b) Verfahrensvariante (c):

Ein Gemisch aus 41,0 g (0,104 Mol) des R-Enantiomeren der 2-[3-(2,6-Dichlor-4-trifluormethyl-phenoxy)-phenoxy]-propionsäure, 15,8 g (0,114 Mol) Kaliumcarbonat und 17,4 g (0,104 Mol) Bromessigsäureethylester in 104 ml Acetonitril wurde 1 Stunde unter Rückfluß erhitzt. Danach wurde aufgearbeitet, indem man das Reaktionsgemisch absaugte, das Filtrat einengte, den verbliebenen Rückstand in Methylenchlorid aufnahm, diese Lösung einmal mit Wasser wusch und nach dem Trocknen einengte. Der verbleibende Rückstand wurde durch leichtes Erwärmen im Hochvakuum von Resten an flüchtigen Anteilen befreit. Man erhielt auf diese Weise 34,9 g (70 % der Theorie) an R-Enantiomerem des 2-[3-(2,6-Dichlor-4-trifluormethyl-phenoxy)-phenoxy]-propionsäure-(ethoxycarbonyl)-methylesters in Form einer Flüssigkeit mit dem Brechungsindex $n_D^{20}$ = 1,5190.

Drehwert: $[\alpha]_D^{24}$ = +4,68°
(1-molare Lösung in Chloroform; Küvettenlänge 10 cm).

In ein Gemisch von 8,2 g Essigsäureanhydrid, 0,5 g Wasser und 4,83 g (0,02 Mol) Kupfernitrat $[Cu(NO_3)_2 \cdot 3H_2O]$ wurde bei 20°C unter Rühren eine Lösung von

9,6 g (0,02 Mol) R-Enantiomerem des 2-/3-(2,6-Dichlor-4-trifluormethyl-phenoxy)-phenoxy7-propionsäure-(ethoxycarbonyl)-methylesters in 6 g Essigsäureanhydrid eingetropft. Es wurde 1,5 Stunden nachgerührt und dann aufgearbeitet, indem man das Reaktionsgemisch in 100 ml Eiswasser goß, das entstehende Gemisch zweimal mit je 100 ml Ether extrahierte, die vereinigten organischen Phasen trocknete und dann durch Abziehen des Lösungsmittels unter vermindertem Druck einengte. Man erhielt auf diese Weise das R-Enantiomere des 2-/3-(2,6-Dichlor-4-trifluormethyl-phenoxy)-phenoxy7-propionsäure-(ethoxycarbonyl)-methylesters in einer Ausbeute von 89 % der Theorie.

Drehwert: $[\alpha]_D^{24}$ = -10,6°
(1-molare Lösung in Chloroform; Küvettenlänge 10 cm).

<u>Herstellung der Ausgangssubstanzen:</u>

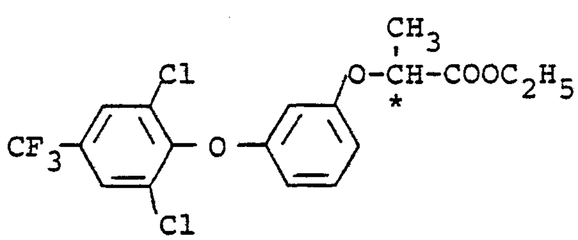

Ein Gemisch aus 96,9 g (0,3 Mol) 2,6-Dichlor-4-trifluormethyl-3'-hydroxy-diphenylether, 81,7 g (0,3 Mol) des S-Enantiomeren des 2-Tosyloxy-propionsäureethylesters und 82,8 g (0,6 Mol) Kaliumcarbonat in 800 ml Acetonitril wurde 3 Stunden unter Rückfluß erhitzt. Danach wurde aufgearbeitet, indem man das Reaktionsgemisch absaugte, das Filtrat einengte, den verbleibenden Rückstand in Methylenchlorid löste, die entstehende Lösung einmal mit Wasser wusch und nach dem Trocknen unter vermindertem Druck einengte. Der verbleibende Rückstand wurde durch leichtes Erwärmen im Hochvakuum von Resten an flüchtigen Anteilen befreit. Man erhielt auf diese Weise das R-Enantiomere des 2-[3-(2,6-Dichlor-4-trifluormethyl-phenoxy)-phenoxy]-propionsäure-ethylesters in einer Ausbeute von 97 % der Theorie.

Drehwert: $[\alpha]_D^{24}$ = +1,5°
(1-molare Lösung in Chloroform; Küvettenlänge 10 cm).

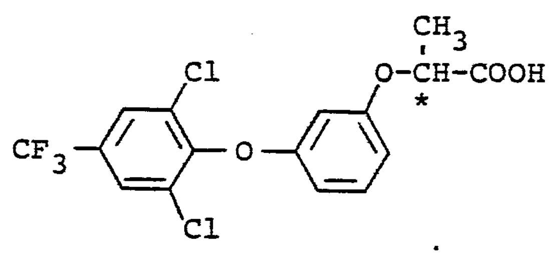

(VII-1)

Eine Lösung von 51,4 g (0,124 Mol) des R-Enantiomeren des 2-[3-(2,6-Dichlor-4-trifluormethyl-phenoxy)-phenoxy]-propionsäure-ethylesters in 95 ml Ethanol wurde unter Rühren bei 0°C in eine Lösung von 5,9 g (0,148 Mol) Natriumhydroxid in 145 ml Wasser eingetropft. Dabei schäumte das Reaktionsgemisch so stark, daß nach Zugabe von 2/3 der Ester-Lösung zunächst noch 10 ml Ethanol in das Reaktionsgemisch gegeben werden mußten, bevor der Rest der Ester-Lösung hinzugefügt werden konnte. Man rührte noch weitere 16 Stunden unter Eiskühlung und

arbeitete dann auf, indem man mit Salzsäure ansäuerte, das entstehende Gemisch dreimal mit Methylenchlorid extrahierte und nach dem Trocknen einengte. Der verbliebene Rückstand wurde mit Petrolether gekocht und heiß abgesaugt. Man erhielt auf diese Weise 47,6 g (97,1 % der Theorie) an dem R-Enantiomeren der 2-[3-(2,6-Dichlor-4-trifluormethyl-phenoxy)-phenoxy]-propionsäure in Form einer Festsubstanz vom Schmelzpunkt 120°C.

Drehwert: $[\alpha]_D^{24}$ = -1,9°
(1-molare Lösung in Chloroform; Küvettenlänge 10 cm).

Nach den oben angegebenen Methoden wurden auch die in den folgenden Beispielen aufgeführten erfindungsgemäßen Stoffe hergestellt:

<u>Beispiel 2</u>

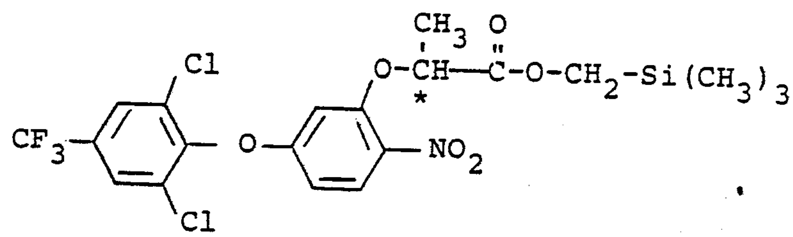

(I-2)

Drehwert: $[\alpha]_D^{24}$ = - 10,9°
(1-molare Lösung in Chloroform; Küvettenlänge 10 cm).

Beispiel 3

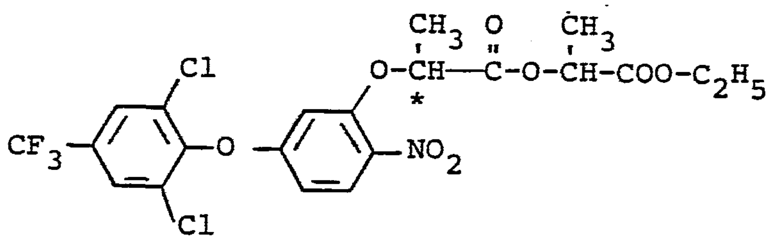

(I-3)

Drehwert: $[\alpha]_D^{24}$ = - 19,85°
R/S-Verhältnis : 88:12
(1-molare Lösung in Chloroform, Küvettenlänge 10 cm).

Beispiel 4

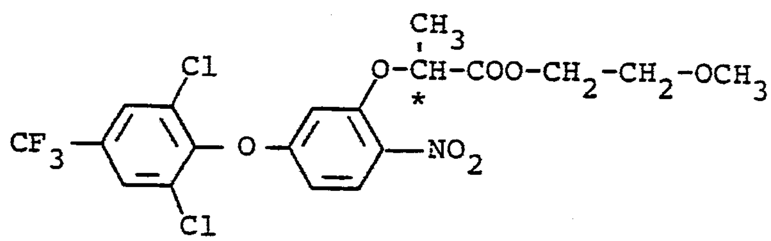

(I-4)

Drehwert: $[\alpha]_D^{24}$ = - 16,23°
R/S-Verhältnis : 91:9
(1-molare Lösung in Chloroform; Küvettenlänge 10 cm).

Beispiel 5

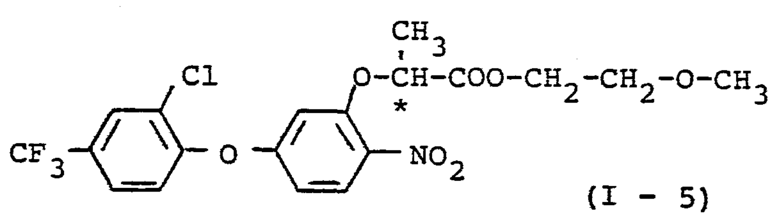

(I - 5)

Drehwert: $[\alpha]_D^{24}$ = - 14,20°
R/S-Verhältnis : 94 : 6
(1-molare Lösung in Chloroform; Küvettenlänge 10 cm).

Beispiel 6

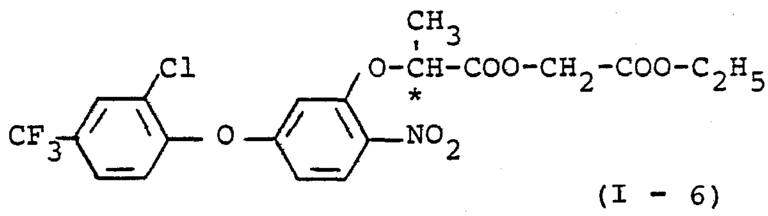

(I - 6)

Drehwert: $[\alpha]_D^{24}$ = - 10,35°
R/S-Verhältnis = 93:7
(1-molare Lösung in Chloroform; Küvettenlänge 10 cm).

Beispiel 7

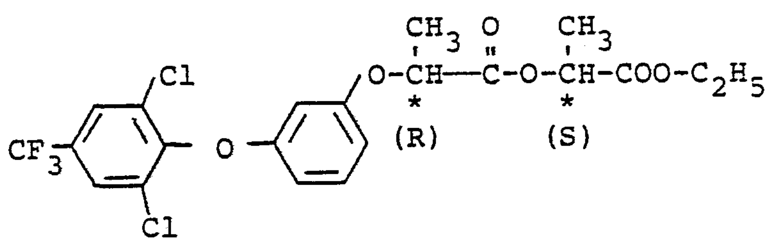

(VI-2)

Ein Gemisch aus 16,1 g (0,05 Mol) 2,6-Dichlor-4-trifluormethyl-3'-hydroxy-diphenylether, 8,2 g (0,06 Mol) Kaliumcarbonat, 17,2 g (0,05 Mol) des Tosylates der Formel

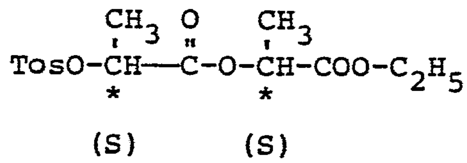

(V-1)

und 100 ml Acetonitril wurde 10 Stunden unter Rückfluß erhitzt. Danach wurde das Reaktionsgemisch abgekühlt, mit 200 ml Wasser versetzt und zweimal mit je 100 ml Methylenchlorid extrahiert. Die vereinigten organischen Phasen wurden über Natriumsulfat getrocknet und dann durch Abziehen des Lösungsmittels unter vermindertem Druck eingeengt. Der verbleibende Rückstand wurde durch leichtes Erwärmen im Hochvakuum von Resten an flüchtigen Anteilen befreit. Man erhielt auf diese Weise 17 g (68 % der Theorie) an der oben bezeichneten Verbindung der Formel (VI-2).
Brechungsindex: $n_D^{20} = 1,5292$

Drehwert: $[\alpha]_D^{24} = -5,2°$
(1-molare Lösung in Chloroform; Küvettenlänge 10 cm).

Nach der im Beispiel 7 angegebenen Methode wurden auch die in den folgenden Beispielen aufgeführten Zwischenprodukte der Formel (VI) hergestellt:

<u>Beispiel 8</u>

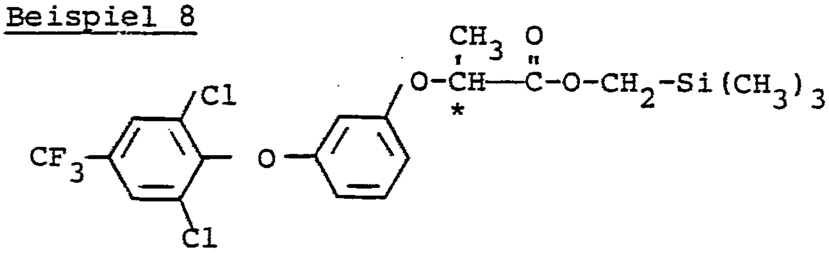

(VI-3)

Drehwert: $[\alpha]_D^{24}$ = +4,3°

Beispiel 9

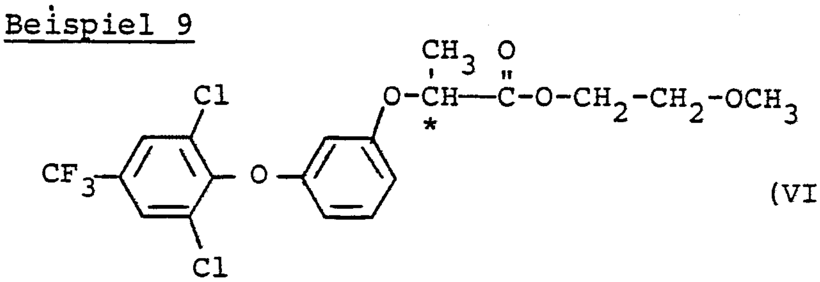

(VI-4)

Drehwert: $[\alpha]_D^{24}$ = +1,7°

Beispiel 10

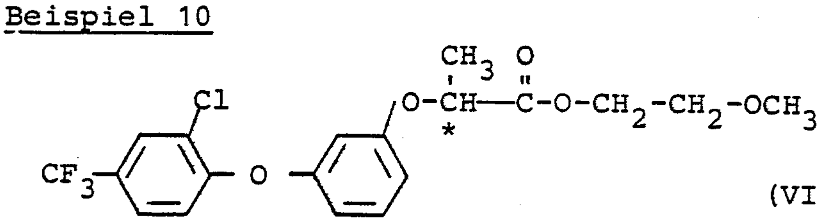

(VI-5)

Drehwert: $[\alpha]_D^{24}$ = +3,45°

Beispiel 11

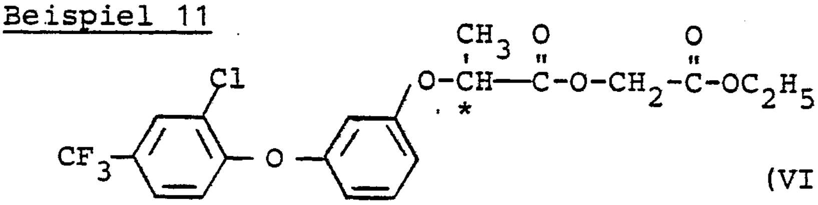

(VI-6)

Drehwert: $[\alpha]_D^{24}$ = +6,72°

Beispiel 12

Herstellung des Ausgangsproduktes der Formel

$$\begin{array}{l} \quad\quad\;\; CH_3 \quad O \\ \quad\quad\quad\; \vert \quad\;\; \Vert \\ TosO-\underset{*}{CH}-C-O-CH_2-COO-C_2H_5 \end{array} \qquad (V-1)$$

(S)

247 g (0,94 Mol) des S-Enantiomeren des Milchsäurechlorid-tosylates wurden bei 10 °C unter Rühren in ein Gemisch aus 98 g (0,94 Mol) Hydroxyessigsäureethylester und 94 g (0,94 Mol) Triethylamin in 300 ml Toluol gegeben. Man rührte weitere 14 Stunden bei 20 °C und arbeitete dann auf, indem man das Reaktionsgemisch mit 600 ml Wasser versetzte und die Toluolphase abtrennte. Nach dem Abziehen des Lösungsmittels unter vermindertem Druck erhielt man 285 g (93 % der Theorie) an dem S-Enantiomeren des 2-Tosyloxy-propionsäure-(ethoxycarbonyl)-methylesters.

Drehwert: $[\alpha]_D^{24}$ = -11,6°
(1-molare Lösung in Chloroform; Küvettenlänge 10 cm).

Nach der im Beispiel 12 angegebenen Methode wurden auch die in den folgenden Beispielen aufgeführten Ausgangsstoffe der Formel (V) hergestellt.

Beispiel 13

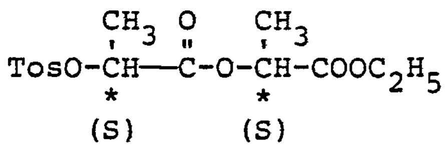

(V-2)

Drehwert: $[\alpha]_D^{24}$ = -17,8°

Beispiel 14

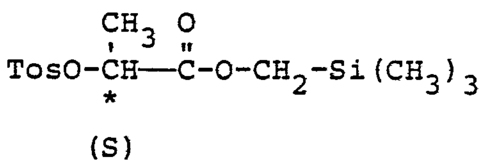

(V-3)

Drehwert: $[\alpha]_D^{24}$ = -11,4°

Beispiel 15

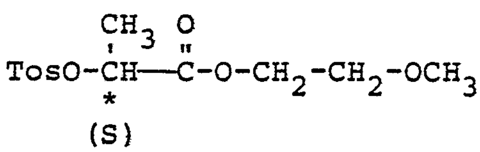

(V-4)

Drehwert: $[\alpha]_D^{24}$ = - 10,49°
Brechungsindex: $n_D^{20}$ = 1,4983

In den folgenden Verwendungsbeispielen wurden die nachstehend aufgeführten Verbindungen eingesetzt:

(A) = 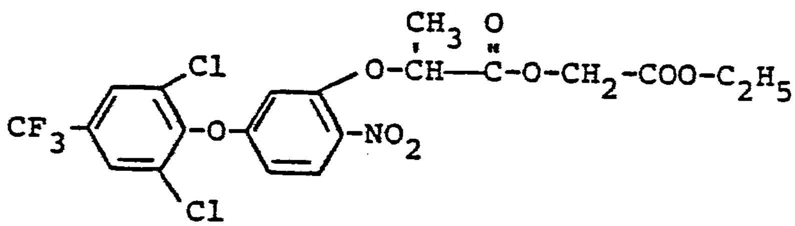

Racemat

(bekannt aus DE-OS 29 06 087)

(I-1) 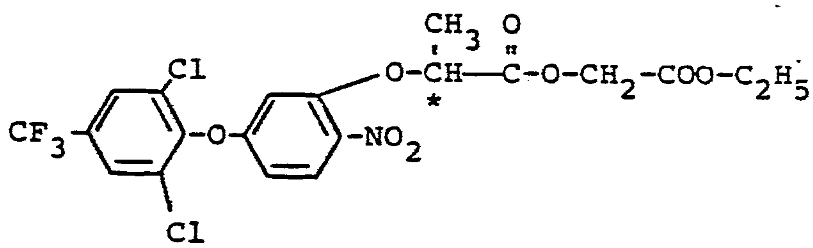

(R-Enantiomeres)

(erfindungsgemäß)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Wirkstoffe, Wirkstoffkonzentrationen und Versuchsergebnisse gehen aus der folgenden Tabelle hervor.

Tabelle A

Post-emergence-Test

| Wirkstoff | Aufwandmenge in kg/ha | Wirkung bzw. Schädigung bei Weizen | Amaranthus | Galium | Polygonum | Gerste |
|---|---|---|---|---|---|---|
| (A) | 0,05 | 10 | 100 | 80 | 80 | 0 |
| (bekannt) | 0,025 | 0 | 100 | 80 | 80 | 0 |
| (I-1) | 0,036 | 10 | 100 | 100 | 100 | 0 |
| (erfindungsgemäß) | 0,018 | 0 | 100 | 90 | 100 | 0 |

Patentansprüche

1. R-Enantiomere von Phenoxypropionsäureestern der Formel

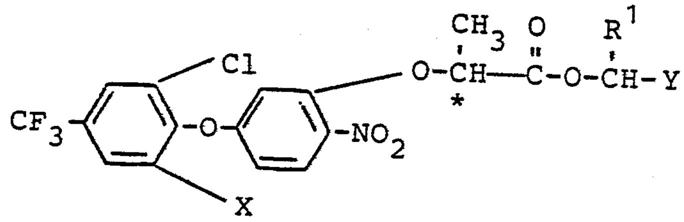

(I)

in welcher

X für Wasserstoff oder Halogen steht,

$R^1$ für Wasserstoff oder Alkyl steht und

Y für Trialkylsilyl, Alkoxyalkyl oder den Rest der Formel $-COOR^2$ steht, worin $R^2$ für Wasserstoff, Alkyl oder Alkoxyalkyl steht.

2. R-Enantiomere von Phenoxypropionsäureestern der Formel (I), in denen

X für Wasserstoff oder Chlor steht,

$R^1$ für Wasserstoff oder Alkyl mit 1 bis 4 Kohlenstoffatomen steht und

Y für Trialkylsilyl mit 1 bis 4 Kohlenstoffatomen in jeder Alkylgruppe, Alkoxyalkyl mit 1 bis 4 Kohlenstoffatomen in der Alkoxygruppe und 1 bis 4 Kohlenstoffatomen in der Alkylgruppe oder für den Rest der Formel $-COOR^2$ steht, worin

$R^2$ für Wasserstoff, Alkyl mit 1 bis 4 Kohlenstoffatomen oder für Alkoxyalkyl mit 1 bis 4 Kohlenstoffatomen in der Alkoxygruppe und 1 bis 4 Kohlenstoffatomen in der Alkylgruppe steht.

3. R-Enantiomeres des Phenoxypropionsäureesters der Formel

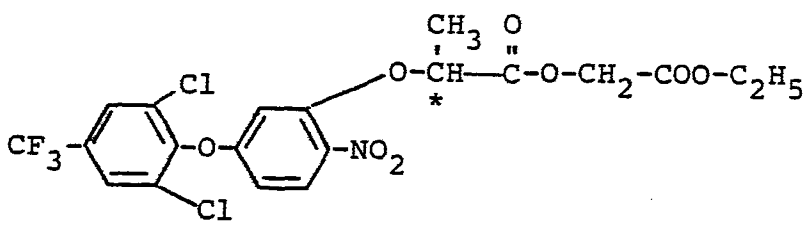

4. Verfahren zur Herstellung von R-Enantiomeren von Phenoxypropionsäure-estern der Formel

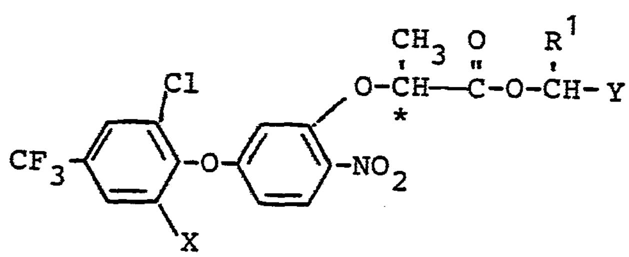

(I)

in welcher

X für Wasserstoff oder Halogen steht,

$R^1$ für Wasserstoff oder Alkyl steht und

Y für Trialkylsilyl, Alkoxyalkyl oder den Rest der Formel $-COOR^2$ steht, worin $R^2$ für Wasserstoff, Alkyl oder Alkoxyalkyl steht,

dadurch gekennzeichnet, daß man

a) R-Enantiomere von Phenoxypropionsäure-Chloriden der Formel

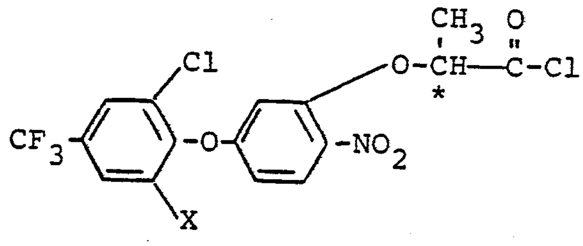

(II)

in welcher

X die oben angegebene Bedeutung hat,

mit Hydroxy-Verbindungen der Formel

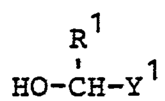

(III)

in welcher

$R^1$ die oben angegebene Bedeutung hat und

$Y^1$ für Trialkylsilyl, Alkoxyalkyl oder den Rest der Formel $-COOR^3$ steht, worin

$R^3$ für Alkyl oder Alkoxyalkyl steht,

gegebenenfalls in Gegenwart eines Säureakzeptors sowie gegebenenfalls in Gegenwart eines Verdünnungsmittels umsetzt, und gegebenenfalls dabei entstehende Ester in Gegenwart eines Verdünnungsmittels verseift,

oder

b) Diphenylether-Derivate der Formel

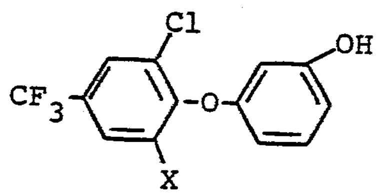

(IV)

in welcher

X die oben angegebene Bedeutung hat

mit S-Enantiomeren der Propionsäure-Derivate der Formel

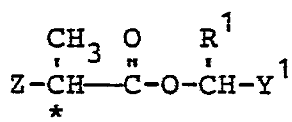

(V)

in welcher

$R^1$ und $Y^1$ die oben angegebene Bedeutung haben und

Z für Tosylat oder Mesylat steht,

gegebenenfalls in Gegenwart eines Säureakzeptors sowie gegebenenfalls in Gegenwart eines Verdünnungsmittels umsetzt und anschließend die dabei entstehenden R-Enantiomeren der Diphenylether-Derivate der Formel

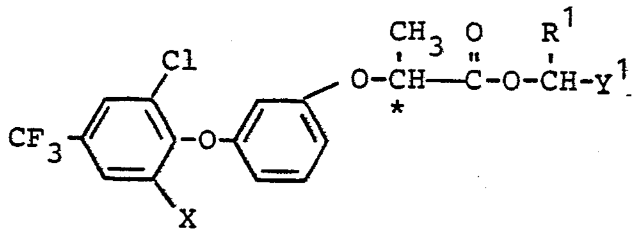

(VI)

in welcher

$R^1$, X und $Y^1$ die oben angegebene Bedeutung haben,

mit einem Nitrierungsmittel, gegebenenfalls in Gegenwart eines Katalysators sowie gegebenenfalls in Gegenwart eines Verdünnungsmittels umsetzt und gegebenenfalls dabei entstehende Ester in Gegenwart eines Verdünnungsmittels verseift,

oder

c) R-Enantiomere von Phenoxypropionsäure-Derivaten der Formel

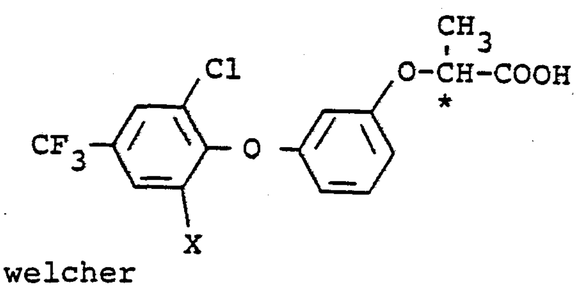

(VII)

in welcher

X die oben angegebene Bedeutung hat,

mit Halogen-Verbindungen der Formel

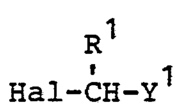

(VIII)

in welcher

$R^1$ und $Y^1$ die oben angegebene Bedeutung haben und

Hal für Chlor oder Brom steht,

in Gegenwart eines Säureakzeptors sowie gegebenenfalls in Gegenwart eines Verdünnungsmittels umsetzt und anschließend die dabei entstehenden R-Enantiomeren der Diphenylether-Derivate der Formel

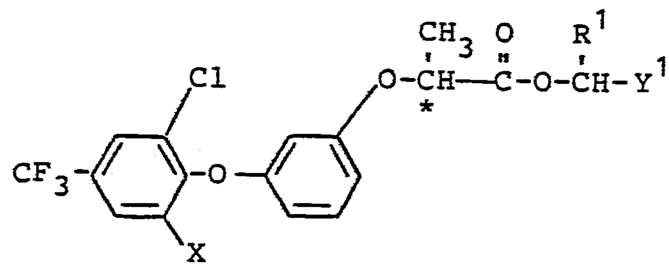

(VI)

in welcher

$R^1$, X und $Y^1$ die oben angegebene Bedeutung haben,

mit einem Nitrierungsmittel, gegebenenfalls in Gegenwart eines Katalysators sowie gegebenenfalls in Gegenwart eines Verdünnungsmittels umsetzt und gegebenenfalls dabei entstehende Ester in Gegenwart eines Verdünnungsmittels verseift.

5. Herbizide Mittel, gekennzeichnet durch einen Gehalt an mindestens einem R-Enantiomeren eines Phenoxypropionsäure-esters der Formel (I).

6. Verwendung von R-Enantiomeren von Phenoxypropionsäure-estern der Formel (I) als Herbizide.

7. Verfahren zur Bekämpfung von Unkräutern, dadurch gekennzeichnet, daß man R-Enantiomere von Phenoxypropionsäure-estern der Formel (I) auf die Unkräuter und/oder deren Lebensraum ausbringt.

8. Verfahren zur Herstellung von herbiziden Mitteln, dadurch gekennzeichnet, daß man R-Enantiomere von Phenoxypropionsäure-estern der Formel (I) mit Streckmitteln und/oder oberflächenaktiven Stoffen vermischt.

9. R-Enantiomeres des Phenoxypropionsäure-esters der Formel

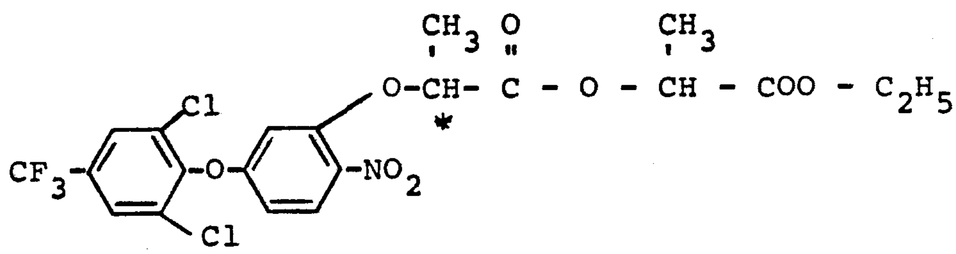

1o. R-Enantiomeres des Phenoxypropionsäure-esters der Formel

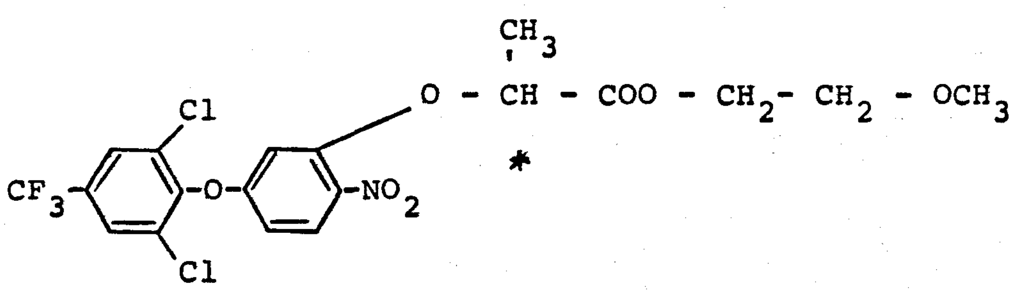

Europäisches Patentamt

EUROPÄISCHER RECHERCHENBERICHT

0128381
Nummer der Anmeldung

EP 84 10 5433

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | EP-A-0 014 900 (BAYER)<br>* Patentansprüche; Tabelle 1 *<br>--- | 1-10 | C 07 C 79/355<br>C 07 F 7/08<br>A 01 N 39/02<br>A 01 N 55/00 |
| Y | EP-A-0 077 962 (BAYER)<br>* Patentansprüche; Beispiel 2 *<br>--- | 1-8 | |
| Y | EP-A-0 001 641 (CIBA-GEIGY)<br>* Patentansprüche; Beispiel 2 *<br>--- | 1-8,10 | |
| Y | EP-A-0 002 800 (HOECHST)<br>* Patentansprüche; Beispiele 6B,21,26; Seite 2, Zeilen 8-10 *<br>--- | 1-8 | |
| P,Y | EP-A-0 095 115 (BAYER)<br>* Patentansprüche; Beispiel 8 *<br>----- | 1-8 | |
| | | | **RECHERCHIERTE SACHGEBIETE (Int. Cl. 3)** |
| | | | C 07 C 79/00<br>C 07 F 7/00 |

Der vorliegende Recherchenbericht wurde für alle Patentansprüche erstellt.

| Recherchenort | Abschlußdatum der Recherche | Prüfer |
|---|---|---|
| DEN HAAG | 30-08-1984 | WRIGHT M.W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument